# EUROPÄISCHE PATENTSCHRIFT

(11) **EP 0 697 225 B1**
(45) Veröffentlichungstag und Bekanntmachung des Hinweises auf die Patenterteilung: **03.05.2000**
(21) Anmeldenummer: 95110963.6
(22) Anmeldetag: 13.07.1995
(51) Int. Cl.: A61M 16/08, A62B 9/00

(54) **Gaszuführleitung**
Gas feed conduit
Conduit d'admission de gaz

(30) Priorität: 22.07.1994 DE 9411933 U
(43) Veröffentlichungstag der Anmeldung: 21.02.1996
(73) Patentinhaber: GOTTLIEB WEINMANN GERÄTE FÜR MEDIZIN UND ARBEITSSCHUTZ GMBH & CO., 22525 Hamburg (DE)
(72) Erfinder: Ebers, Manfred, D-22457 Hamburg (DE)
(74) Vertreter: Liebelt, Rolf, Dipl.-Ing.

(56) Entgegenhaltungen:
- EP-A- 0 108 552
- US-A- 4 320 754
- US-A- 4 558 708

## Beschreibung

Die Erfindung betrifft eine Gaszuführleitung, die einen Abschnitt, über den Gas an die Umgebung abgegeben wird, aufweist und an eine Atemmaske zur künstlichen Beatmung eines Patienten anschließbar ist.

Bei der künstlichen Beatmung eines Patienten ist ein Beatmungsgerät über die Gaszuführleitung mit einer Atemmaske verbunden. Diese Leitung weist Bohrungen auf, über die die Überschußluft und die vom Patienten ausgeatmeten Gase an die Umgebung abgegeben werden. Diese Bohrungen sind möglichst nahe zur Atemmaske in der Gaszuführleitung vorgesehen, damit die gesamte Menge der ausgeatmeten Gase nicht bis in das Beatmungsgerät zurückströmt. Das Abführen der ausgeatmeten Atemgase über derartige Bohrungen wird bei häufiger und lang andauernder künstlicher Beatmung, z. B. bei der Behandlung einer Schlafapnoe wegen der entstehenden Geräusche als lästig empfunden. Der aus diesen Bohrungen austretende Luftstrom kann auch Folgeerkrankungen wie Bindehautentzündungen bewirken.

Aufgabe der Erfindung ist es nun, eine patientenfreundliche Ableitung der ausgeatmeten Gase und der Überschußluft bei der künstlichen Beatmung zu schaffen.

Diese Aufgabe wird erfindungsgemäß ausgehend von einer Gaszuführleitung der eingangs beschriebenen Gattung dadurch gelöst, daß der Abschnitt der Gaszuführleitung, über den Gas an die Umgebung abgegeben wird, aus einem porösen oder feinporigen Werkstoff gefertigt ist.

Das ausgeatmete Gas und die Überschußluft werden über den erfindungsgemäß porös oder feinporig ausgebildeten Abschnitt der Gaszuführleitung an die Umgebung abgegeben, ohne daß ein konzentrierter Luftstrom auftritt, da die Luft nach allen Seiten durch die Poren des Materials nach außen strömt. Die dabei entstehenden Geräusche sind in der Regel nicht wahrnehmbar. Es ist auch der Luftstrom außerhalb des porösen Abschnittes, der aus einen Sinterwerkstoff gefertigt und auswechselbar in die Gaszuführleitung eingesetzt sein kann, schon in einen Abstand von wenigen Zentimetern von der Leitung nicht mehr meßbar, wodurch Belästigungen des Patienten bzw. Dritter durch einen konzentrierten Luftstrom vermieden werden.

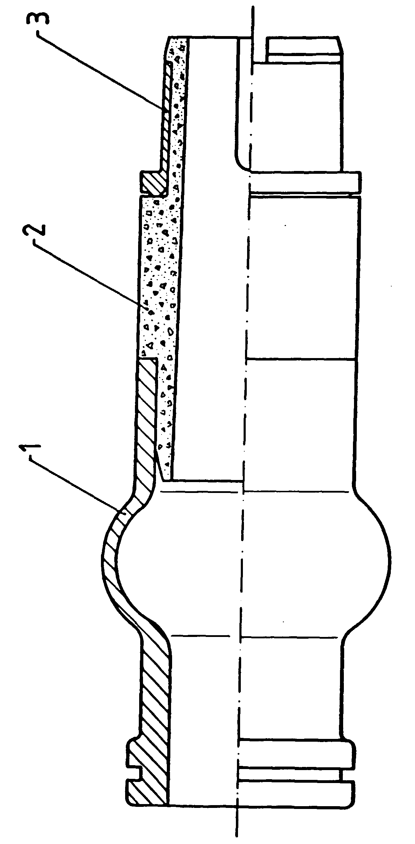

Ein Ausführungsbeispiel der Erfindung wird noch an Hand der Zeichnung beschrieben, in der ein Abschnitt einer Gaszuführleitung zur künstlichen Beatmung eines Patienten in schematischer Teilschnitransicht dargestellt ist.

Der gezeigte Leitungsabschnitt besteht aus einem Gelenkstück 1, das aus einem gummielastischen Material gefertigt und mit dem freien Ende an eine Atemmaske (nicht gezeigt) anschließbar ist. Das andere Ende des Gelenkstückes 1 ist auf einen Abschnitt 2 der Gaszuführleitung gesteckt. Dieser Abschnitt 2 ist aus einem porösen Material gefertigt, durch das das ausgeatmete Gas und Überschußluft nach außen in die Umgebung entweichen kann. Auf das dem Gelenkstück 1 gegenüberliegende Ende des Abschnittes 2 ist eine drehbar gehaltene Hülse 3 aufgeschnappt, auf der ein mit einem Beatmungsgerät verbundener und nicht dargestellter Schlauch anordenbar ist.

## Patentansprüche

1. Gaszuführleitung, die einen Abschnitt, über den Gas an die Umgebung abgegeben wird, aufweist und an eine Atemmaske zur künstlichen Beatmung eines Patienten anschließbar ist, dadurch gekennzeichnet, daß der Abschnitt (2) aus einen porösen oder feinporigen Werkstoff besteht.

2. Gaszuführleitung nach Anspruch 1, dadurch gekennzeichnet, daß der poröse oder feinporige Abschnitt (2) auswechselbar in die Gaszuführleitung eingefügt ist.

3. Gaszuführleitung nach Anspruch 1 oder 2, dadurch gekennzeichnet, daß der Abschnitt (2) aus eines Sintermaterial gefertigt ist.

## Claims

1. Gas feed conduit with a section via which gas is released into the surroundings and which can be connected to a breathing mask for the artificial respiration of a patient, characterised in that the section (2) consists of a porous or fine-pored material.

2. Gas feed conduit according to claim 1, characterised in that the porous or fine-pored section (2) is inserted removably into the gas feed conduit.

3. Gas feed conduit according to claim 1 or 2, characterised in that the section (2) is produced from a sintered material.

## Revendications

1. Conduit d'admission de gaz, comportant une portion qui délivre du gaz en périphérie et qui peut être raccordée à un masque respiratoire pour la respiration artificielle d'un patient, caractérisé en ce que la portion (2) est constituée d'un matériau poreux ou microporeux.

2. Conduit d'admission de gaz selon la revendication 1, caractérisé en ce que la portion poreuse ou microporeuse (2) est insérée de façon amovible dans ledit conduit d'admission de gaz.

3. Conduit d'admission de gaz selon la revendication 1 ou 2, caractérisé en ce que la portion (2) est fabriquée à partir d'un matériau fritté.
